# EUROPEAN PATENT SPECIFICATION

(11) **EP 3 621 587 B1**
(45) Date of publication and mention of the grant of the patent: **14.07.2021**
(21) Application number: 18716969.3
(22) Date of filing: 03.04.2018
(51) Int. Cl.: A61K 8/81, A61Q 5/06

(54) **HAIR STYLING COMPRISING POLYQUATERNIUM-4,POLYQUATERNIUM-37 AND C12-15 ALKYL BENZOATE**
HAARSTYLING MIT POLYQUATERNIUM-4, POLYQUATERNIUM-37 UND C12-15-ALKYLBENZOAT
COMPOSITION DE COIFFURE COMPRENANT DU POLYQUATERNIUM-4, DU POLYQUATERNIUM-37 ET DU BENZOATE D'ALKYLE C12-15

(30) Priority: 12.05.2017 WO PCT/CN2017/084151
(43) Date of publication of application: 18.03.2020
(73) Proprietor: Beiersdorf AG, 20253 Hamburg (DE)
(72) Inventor: CAO, Mi, Wuhan 430073 (CN)
(86) International application number: PCT/EP2018/058442
(87) International publication number: WO 2018/206198

(56) References cited:
- EP-A1- 1 997 472
- WO-A1-2007/135083
- "Hair conditioners based on new biodegradable cationics", RESEARCH DISCLOSURE, KENNETH MASON PUBLICATIONS, HAMPSHIRE, UK, GB, vol. 531, no. 19, 1 July 2008 (2008-07-01) , page 593, XP007138308, ISSN: 0374-4353
- ANONYMOUS: "Preparation of hair conditioner formulations by a cold process", RESEARCH DISCLOSURE, KENNETH MASON PUBLICATIONS, HAMPSHIRE, UK, GB, vol. 476, no. 4, 1 December 2003 (2003-12-01), XP007133172, ISSN: 0374-4353
- DATABASE GNPD [Online] MINTEL; March 2017 (2017-03), "Superbly Restoring Hair Repair Treatment", XP002781043, Database accession no. 4714501
- DATABASE GNPD [Online] MINTEL; January 2012 (2012-01), "Smoothing Blow Dry Cream", XP002781044, Database accession no. 1691984

## Description

### Technical field

The present invention belongs to the cosmetic field and relates to a hair styling composition for human hair comprising Polyquaternium-4, Polyquaternium-37 and C12-15 Alkyl Benzoate. It was surprisingly found that upon use of the cosmetic hair styling composition shine is provided to the hair. Furthermore, the cosmetic hair styling composition improved the smoothness of the hair after the volatile parts of the cosmetic hair styling composition have evaporated.

### Technical background

Cosmetic hair styling products such as hairsprays, creams, mousses, gels, lotions or waxes allow the customer to temporarily design and style their hair on the scalp in many individual and original ways. The use of the different product forms listed above depends thereby on the regional popularity as well as the desired result. For example, hairsprays are most often used to fix the position of pre-aligned hair fibers while the other products are usually distributed through the hair before shaping or forming the style. In both cases, the cosmetic compositions contain styling polymers which allow for an inter-fiber adhesion.

Today, there is a huge variety of styling products commercially available, claiming effects such as improved hair volume, improved hold or less frizz. Nevertheless, this fact should not obscure that today's products still suffer a number of disadvantages and, therefore, it still remains desirable to improve the product properties further.

One aspect, which should be mentioned in particular, is that hair styling compositions often do not provide sufficiently shine to the human hair. To address this issue a number of approaches have been presented.

For example, the document WO 2007075969 A1 discloses a composition containing a volatile silicone, a hair fixation resin, which is a styling polymer, and an organic solvent. However, the document is entirely silent on the use of a combination such as disclosed in this document.

W2012168035 A1 discloses cosmetic styling composition containing certain anionic polymers which provide the hair with an improved shine. The example compositions presented do not contain a cationic polymer.

WO2007135083 A1 discloses further hair care compositions comprising Polyquaternium-4 and Polyquaternium-37. However, the compositions do not contain C12-15 alkyl benzoate.

EP 1997472 A1 discloses further cosmetic hair compositions comprising Polyquaternium-4 and Polyquaternium-37. However, the compositions do not contain C12-15 alkyl benzoate.

Furthermore, the person skilled in art is aware of the products "Superbly Restoring Hair Repair Treatment", disclosed under the Mintel GNPD record 471501, and "Smoothing Blow Dry Cream", disclosed under the Mintel GNPD record 1691984. Both products

Furthermore, the person skilled in art is aware of the research disclosures "Preparation of hair conditioner formulations by a cold process", Kenneth Mason Publications, Hampshire, UK, GB, vol. 476, no. 4, 1st of December 2003; and "Hair conditioners based on new biodegradable cations" Kenneth Mason Publications, Hampshire, UK, GB, vol. 531, no. 19, 1st of July 2008.

A second aspect, which should also be mentioned in particular, is that after application of the styling products, the hair suffers from an insufficient smoothness after drying.

### Summary

The objective of the present invention is therefore, to provide a hair styling composition, which allows to provide shine to the human hair and/or which improves the smoothness of the hair after drying.

The applicant has surprisingly discovered that a cosmetic hair styling composition comprising in a cosmetically acceptable medium Polyquaternium-4, Polyquaternium-37 and C12-15 Alkyl Benzoate allows to provide shine to the hair and to improve the smoothness of the hair after the solvent has evaporated.

The term "hair" as used throughout the Description refers solely to human hair on the scalp.

All the weight percentages (% by weight) given below relate, unless otherwise stated, to the total weight of the cosmetic hair styling composition.

Unless otherwise stated, all tests were performed under "normal conditions". The term "normal conditions" means 20°C, 1013 hPa and a relative humidity of 50%.

For the purposes of the present disclosure, the term "free from" means that the proportion of the respective substance is less than 0.05% by weight. This ensures that entrainments or impurities with these substances are not included as "free from" according to the invention.

According to the invention the cosmetic hair styling composition contains Polyquaternium-4. Polyquaternium-4 is a polymeric quaternary ammonium salt of hydroxyethylcellulose quarternized with diallyldimethyl ammonium chloride.

Thereby, it is preferred, if the Polyquaternium-4 is therein characterized that it has a viscosity of 20 to 400 cps, when it is suspended as 2% solution in water. The viscosity is determined using a Brookfield LVT Viscometer at 20 RPM, Spindle #2 at 21 °C.

Furthermore, it is also preferred, if the Polyquaternium-4 is therein characterized that the content of Nitrogen is 1.5% to 3% by weight of the Polyquaternium-4.

A typical Polyquaternium-4 fulfilling the requirements above is commercially marketed under the trade name Celquat® L-200 by the company AkzoNobel.

The concentration of Polyquaternium-4 in the cosmetic hair styling composition of the invention is preferably in the range from 0.1% to 2% by weight and more preferably in the range from 0.5% to 1% by weight, calculated to the total weight of the composition.

In addition to the Polyquaternium-4 the cosmetic hair styling composition contains Polyquaternium-37.

Polyquaternium-37 is a cationic homopolymer comprising as repetitive monomeric unit of a constituent according to formula (I) 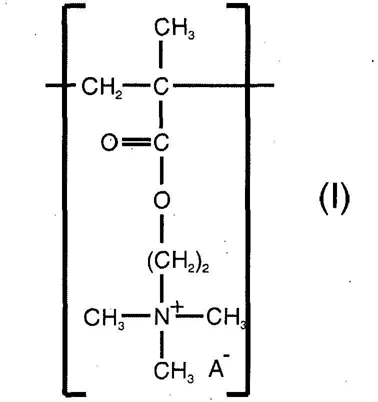 wherein A represents chloride.

In many cases Polyquaternium-37 is supplied as emulsion containing an oil and an emulsifier. Such a mixture, containing mineral oil as oil and Trideceth-6 as emulsifier is sold under the trade name Structure PQ-37M by AkzoNoble. Other commercial products contain, for example, Propylene Glycol Dicaprylate Dicaprate and PPG-1 Trideceth-6 (for example Salcare SC95, sold by BASF). However, even though Polyquaternium-37 is supplied in the most cases as emulsion, it was found that it is advantageous to suspend Polyquaternium-37 in solid form directly in the cosmetic composition instead of using a pre-emulsified Polyquaternium-37 market product. This measure excludes the fact that the emulsifiers and oils contained in the pre-emulsion influence the final product properties.

Polyquaternium-37 in form of a powder can commercially by purchased under the trade name Cosmedia® Ultragel 300 from the company BASF.

Preferably the concentration of Polyquaternium-37 in the cosmetic hair styling composition is in the range from 0.1% to 2% by weight, more preferably in the range from 0.5% to 1.5% by weight, calculated to the total weight of the composition.

Furthermore, the cosmetic composition of the invention contains C12-15 Alkyl Benzoate. C12-15 Alkyl Benzoate is the CTFA declaration for a mixture of Alkyl Benzoates, wherein the alkyl groups comprise 12 to 15 carbon atoms.

The concentration of C12-C15 Alkyl Benzoate in the cosmetic hair styling composition of the invention is preferably in the range from 0.1% to 6% by weight, more preferably in the range from 1% to 3% by weight, calculated to the total weight of the composition.

C12-C15 Alkyl Benzoate is, for example, commercially marketed under the trade name TEGOSOFT TN B by the company Evonik Industries.

The cosmetically acceptable medium for containing the inventive composition is preferably water. Thereby, the concentration of water in the cosmetic hair styling composition of the invention is preferably in the range from 65% to 95% by weight, more preferably in the range from 80% to 90 % by weight, calculated to the total weight of the composition.

Moreover, in accordance with invention it is preferred if the cosmetic hair styling composition is an O/W-Emulsion.

In order to form a stable O/W-Emulsion, it is further preferred if the cosmetic hair styling composition contains at least one emulsifier having an HLB value in the range from 13 to 17 and more preferably in the range from 15 to 16.

Thereby, it is especially preferred, if the cosmetic hair styling composition of the invention does not contain further emulsifiers with an HLB value outside of the above specified preferred range.

A variety of oil-in-water emulsifiers and their HLB values are compiled in the table below. These are emulsifiers commonly known to one skilled in the art, such as those listed, for example, in Kirk Othmer, "Encyclopedia of Chemical Technology," 3rd ed.,1979, Vol. 8, pp. 913-916.

| HLB value | Chemical designation |
|---|---|
| 12.0 | PEG-10 Soy Sterol (e.g., Generol 122 E 10) |
| | Triethanolfillline oleate |
| 12.2 | Nonylphenol, ethoxylated with 8 mol EO |
| | Sucrose monomyristate |
| 12.4 | Sucrose monolaurate |
| | Polyoxyethylene (10) oleyl alcohol, polyoxyethylene (10) oleyl ether |
| | Polyoxyethylene (10) stearyl alcohol, polyoxyethylene (10) stearyl ether |
| 12.5 | Polyoxyethylene (10) stearyl cetyl ether |
| 12.7 | Polyoxyethylene (8) tridecyl alcohol |
| 12.8 | Polyoxyethylene glycol (400) monolaurate |
| | Sucrose Monococoate |
| 12.9 | Polyoxyethylene (10) cetyl ether |
| 13.0 | Glyceryl Monostearate, ethoxylated (20 mol EO) |
| | Polyoxyethylene (10) oleyl ether (Eumulgin O 10) |
| | Nonoxynol-10 (Eumulgin 286) |
| | Ceteareth-12 (Eumulgin B1 |
| | C12 fatty amines, ethoxylated (5 mol EO) |
| 13.1 | Nonylphenol, ethoxylated (9.5 mol EO) |
| 13.2 | Polyethylene glycol (600) monostearate |
| | Polyoxyethylene (16) tall oil |
| 13.3 | Polyoxyethylene (4) sorbitan monolaurate |
| 13.5 | Nonylphenol, ethoxylated (10.5 mol EO) |
| | Polyethylene glycol (600) monooleate |
| 13.7 | Polyoxyethylene (10) tridecyl alcohol |
| | Polyethylene glycol (660) monotallate |
| | Polyethylene glycol (1500) monostearate |
| | Polyoxyethylene glycol (1500) dioleate |
| 13.9 | Polyethylene glycol (400) monococoate |
| | Polyoxyethylene (9) monolaurate |
| 14.0 | Polyoxyethylene (12) lauryl ether |
| | Polyoxyethylene (12) tridecyl alcohol |
| 14.2 | Polyoxyethylene (15) stearyl alcohol |
| 14.3 | Polyoxyethylene (15) stearylcetyl ether |
| 14.5 | Polyoxyethylene (12) lauryl alcohol |
| 14.8 | Polyethylene glycol (600) monolaurate |
| 14.9 | Sorbitan monostearate, ethoxylated with 20 mol EO (Emulgin SMS 20) |
| 15.0 | Sorbian monooleate, ethoxylated with 20 mol EO (Emulgin SMO 20) |
| | PEG-20 glyceryl stearate |
| | PEG-40 Castor Oil |
| | Decyl glucoside |
| | Dodecyl glycoside |
| | Nonylphenol, ethoxylated with 15 mol EO |
| | Polyethylene glycol (1000) monostearate |
| | Polyoxyethylene (600) monooleate |
| 15.3 | Polyoxyethylene (20) oleyl alcohol, polyoxyethylene (20) oleyl ether |
| 15.4 | Polyoxyethylene (20) stearyl cetyl ether |
| 15.5 | Polyoxyethylene (20) stearyl alcohol |
| 15.6 | Polyoxyethylene glycol (1000) monostearate |
| | Polyoxyethylene (20) sorbitan monopalmitate |
| 15.7 | Polyoxyethylene (20) cetyl ether |
| 16.0 | Polyoxyethylene (25) propylene glycol stearate |
| | Nonylphenol ethoxylated with 20 mol EO |
| 16.6 | Polyoxyethylene (20) sorbitol |
| 16.7 | Polyoxyethylene (23) lauryl alcohol |
| 17 | Ceteareth-30 |
| | Polyoxyethylene (30) glyceryl monolaurate |
| 17.1 | Nonylphenol, ethoxylated with 30 mol EO |
| 17.4 | Polyoxyethylene (40) stearyl alcohol |

In the table above and the following disclosure the term EO stands for ethylene oxide.

Cosmetic hair styling compositions that are extraordinarily preferred according to the present invention contain sorbitan monostearate, ethoxylated with 20 mol EO, and/or sorbian monooleate, ethoxylated with 20 EO. Furthermore, those cosmetic hair styling composition do not contain further emulsifiers.

Sorbitan monostearate, ethoxylated with 20 mol EO, is known under the CTFA declaration Polysorbate 60. Sorbian monooleate, ethoxylated with 20 EO is known under the CTFA declaration Polysorbate 80.

The advantage in using solely Polysorbate 60 and/or Polysorbate 80 is that the cosmetic hair styling composition, which is in form of an O/W-emulsion, has a milky, non-transparent look. It was found that customers prefer to use such products, as due to the milky appearance of the emulsion it is believed that the product provides a better caring and/or conditioning effect. The use of other emulsifiers, such as Ceteareth-20, results in a clearer and more transparent composition.

The cosmetic hair styling composition according to the present invention may further comprise a water insoluble, non-volatile silicone compound. If such silicone compounds are contained in the composition according to the invention, the silicone compounds further benefit the smoothness of the hair. They may further improve the shine of the hair in association with the Polyquaternium Polymers and the C12-15 Alky Benzoate of the present invention.

The term "water-insoluble" is used herein to mean a solubility of less than 50% by weight obtained when 1 part by weight of a substance is dissolved in 99 parts by weight of water at 25°C.

One preferred group of silicone compounds is known under the designation dimethicone. Dimethicones may be linear, branches, cyclic or cyclic and branched. Linear dimethicones may be illustrated by the formula (II) 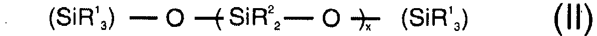 and branched dimethicones may be illustrated by the formula (III) 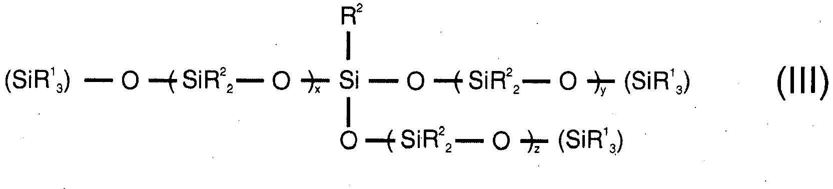 wherein each R¹ and R² are independently selected from a hydrogen atom, a methyl group, a linear or branched, saturated or unsaturated hydrocarbon group containing 2 to 30 carbon atoms, an aryl group and/or an alkylaryl group.

According to the invention it is preferred if R¹ and R² are independently selected from a methyl group, phenyl group and an alkyl group containing 2 to 22 carbon atoms. In the case R¹ and/or R² are selected to be an alkyl group, the alkyl group is preferably a lauryl, stearyl and/or behenyl group. The letters x, y and z in formula above represent independently integer digits in the range from 0 to 60000.

It is most preferred if the dimethicone is a linear Polyalkylsiloxane.

An essential parameter defining the properties of the dimethicone is its viscosity. Preferably the dimethicone, such as the preferred linear polymethylsiloxanes, are characterized therein that they have a viscosity in range from 0.01 to 100000 cPs, more preferably in the range from 10 to 1000 cPs measured at a temperature of 25°C using a glas capillary viscosimeter according to the Dow Corning corporate Test method CTM 0004, version of the July 20, 1970.

Typical examples of commercially available dimethicones include DC 200 Fluid 20 cSt, DC 200 Fluid 100 cSt and DC 200 Fluid 350 cSt from Dow Corning Corporation.

According to the invention it is also possible to include the silicone compound in form of a silicone emulsion. Such a silicone emulsion is preferably therein characterized that the average diameter of the silicon particles is less than 1000 nm, more preferably less than 600 nm. If a silicon emulsion with a particle size of less than 1000 nm, more preferably less than 600 nm is contained, it is advantageous that due to the small particle size the silicone is effectively deposited onto the hair to allow for a caring effect, while the hair is not significantly being weighed down by the silicone compound.

Within the meaning of the present invention the term "average diameter of the silicone particles" is understood to be the volume-average silicone particle diameter D50, which can be determined by laser diffractometry. The term "volume-average particle diameter D50" is the point in the particle size distribution at which 50 vol.% of the silicone particles have a smaller diameter and 50 vol.% of the silicone particles have a larger diameter.

Especially preferred silicone emulsions are further therein characterized that they contain
(i) 30% to 70% by weight, calculated to the total weight of the emulsion, of at least one polyalkylsiloxane, most preferably polymethylsiloxane;
(ii) at least one nonionic emulsifier selected from alkoxylated C8 to C24 alcohols containing 18 to 30 ethylenoxid groups; and
(iii) at least one nonionic emulsifier selected from alkoxylated C8 to C24 alcohols containing 1 to 6 ethylenoxid groups.

Commercial examples of such silicone emulsions are the Xiameter MEM-1352 Emulsion or the Xiameter MEM-1664 Emulsion, both sold by Dow Corning. Both are therein characterized that they have a particle size of less than 600 nm.

The cosmetic hair styling composition according to the invention may further comprise at least one cationic surfactant for solubilizing, antimicrobial and/or further conditioning purposes. Preferably the cationic surfactants are selected from the compounds with the general formula (IV) 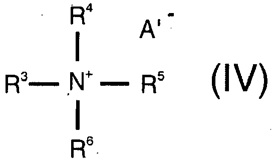
wherein R³ and R⁴ stand for a hydrogen atom or an alkyl chain of 1 to 4 carbon atoms which are optionally substituted with one or more hydroxyl groups,
and wherein R⁵ stands for a saturated or unsaturated, branched or non-branched alky chain of 8 to 24 carbon atoms,
or R⁵ stands for R⁷CON(CH₂)_{y'} group, wherein R⁷ is a saturated or unsaturated, branched or non-branched alkyl chain with 7 to 21 carbon atoms and y' is an integer in the range from 1 to 4,
or R⁵ stands for R⁸COO(CH₂)_{z'} group, wherein R⁸ stands for a saturated or unsaturated, branched or non-branched alkyl chain with 7 to 21 carbon atoms and z' is an integer in the range from 1 to 4,
and wherein R⁶ stands for a hydrogen atom or for a saturated or unsaturated, branched or non-branched alky chain of 8 to 24 carbon atoms,
or R⁶ stands for a R⁷CON(CH₂)_{y'} or R⁸COO(CH₂)_{z'} group, wherein R⁷, R⁸, y' and z' have the same meaning as above,
and A' is an anion, preferably chloride or bromide.

Suitable but not limiting examples of above defined cationic surfactants are octyltrimethylammonium chloride, decyltrimethylammonium chloride, dodecyltrimethylammonium chloride, cetyltrimethylammonium chloride, stearyltrimethylammonium chloride, behenyltrimethylammonium chloride, tetramethylammonium chloride, tetraethylammonium chloride, cetylpyridinium chloride, octyldimethylbenzylammonium chloride, decyldimethylbenzylammonium chloride, stearyldimethylbenzylammonium chloride, didodecyldimethylammonium chloride and dioctadecyldimethylammonium chloride.

The most preferred cationic surfactants to be contained in the cosmetic hair styling composition of the present invention are cetyltrimethylammonium chloride and/or behenyltrimethylammonium chloride. Cetyltrimethylammonium chloride is available under the trade name VARISOFT 300 from the Fa. Evonik Industries or Dehyquart® A-CA from the Fa. BASF. Behenyltrimethylammonium chloride is commercially available under the trade name QUARTAMIN® AB from the Fa. Kao Chemicals.

The cationic surfactants are preferably contained in a concentration in the range from 0.1% to 1.4% by weight, more preferably 0.2% to 0.8% by weight, calculated to the total weight of the composition.

The cosmetic hair styling composition of the present invention may further comprise one or more preservatives. Suitable preservatives to be comprised in the composition of the present invention are for example phenoxyethanol, ethylhexyglycerin, methylparaben, ethylparaben, benzyl alcohol, benzethonium chloride, sodium benzoate and/or sodium salicylate.

The cosmetic hair styling composition of the present invention is preferably contained in a pump dispenser such that the composition can easily be removed from the dispenser and applied to the hair.

The composition according to the invention may also comprise substances commonly contained in hair styling compositions. These are for example UV-filter, dyes, ubiquinone such as coenzyme Q10, vitamins, plant extracts, antioxidants such as oryzanol, and perfumes.

### Examples

The following examples should illustrate the compositions of this invention, without, however, intending to limit the invention to these examples. The numerical values in the examples are percentages by weight, based on the total weight of the preparations.

Hair styling compositions

| Ingredients | | Inv. 1 | Com. 1 |
|---|---|---|---|
| Polysorbate 60 | | 1 | 1 |
| Polyquaternium-37¹ | | 1.5 | 1.5 |
| Behentrimonium Chloride (70% active content)+ Dipropylene Glycol (30 % active content) | | 0.5 | 0.5 |
| Dimethicone | | 2 | 2 |
| C12-15 Alkyl Benzoate³ | | 3.5 | 3.5 |
| Polyquaternium-4² | | 0.8 | |
| PVP (Polyvinylpyrrolidon) | | - | 5 |
| Sodium Salicylate | | 0.12 | 0.12 |
| Sodium Benzoate | | 0.3 | 0.3 |
| Perfume | | q.s. | q.s. |
| Aqua | | ad 100 | ad 100 |
| | | | |
| Evaluation | Shine(dry hair) | 8 | 7.5 |
| | Smoothness (dry hair) | 8 | 7.5 |

| | | | |
|---|---|---|---|
| Com = Comparative example, not according to the invention Inv = Inventive example, according to the invention ¹Cosmedia® Ultragel 300 from BASF ²Celquat® L-200 from AkzoNobel. ³TEGOSOFT TN B from Evonik Industries | | | |

A first study was carried out in order to evaluate the properties of the inventive composition Inv. 1 in comparison to the composition Com. 1, which is not in accordance with the invention. The assessment was carried out by trained hairdressers, who evaluated the state of the hair after applying the hair styling compositions. The test was carried out using hair tresses of Asian black hair.

The hair tresses were washed using a standard shampoo then rinsed off until no substance remained. 1.0 g of the styling composition were evenly spread on the 10g half-dry hair tresses. After drying, the trained panelist evaluated the stiffness by analyzing the force which needed to be applied to bend the hair tresses with the fingers. And the smoothness of the hair is evaluated by touching the hair tresses on the top and then gliding through the hair tresses to the bottom with the hands.

Each parameter was rated with a score value between 1 and 10, whereby 1 means bad, and 10 corresponds to very good. Here, it should be noted that regarding the smoothness a score of 10 meant that the hair was very smooth. A score of 10 for the stiffness means that the hair was very stiff.

A second separate study was performed with the compositions Inv.1 and Com.2 to Com.5. For this study other trained panelist were chosen to evaluate the parameter. Apart from that the procedure was the same as for the first study. The results are shown in the table below. The determined results indicate an offset for the composition Inv.1 in reference to the first study, which is due to different panelist taking part. However, the inventive effect is demonstrated in this set of experiments in the same way as for the first study. The effects shown by the second study indicate a synergism between the three inventive ingredients. In both studies the inventive composition showed outstanding effects.

| Ingredients | | Inv. 1 | Com. 2 | Com. 3 | Com. 4 | Com. 5 |
|---|---|---|---|---|---|---|
| Polysorbate 60 | | 1 | 1 | 1 | 1 | 1 |
| Polyquaternium-37¹ | | 1.5 | 1.5 | 1.5 | - | - |
| Behentrimonium Chloride (70% active content)+ Dipropylene Glycol (30 % active content) | | 0.5 | 0.5 | 0.5 | 0.5 | 0.5 |
| Dimethicone | | 2 | 2 | 2 | 2 | 2 |
| C12-15 Alkyl Benzoate³ | | 3.5 | 3.5 | - | 3.5 | - |
| Polyquaternium-4² | | 0.8 | - | 0.8 | 0.8 | - |
| PVP (Polyvinylpyrrolidon) | | - | - | - | - | - |
| Sodium Salicylate | | 0.12 | 0.12 | 0.12 | 0.12 | 0.12 |
| Sodium Benzoate | | 0.3 | 0.3 | 0.3 | 0.3 | 0.3 |
| Parfume | | q.s. | q.s. | q.s. | q.s. | q.s. |
| Aqua | | ad 100 | ad 100 | ad 100 | ad 100 | ad 100 |
| | | | | | | |
| Evaluation | Shine(dry hair) | 8.5 | 8 | 8.1 | 8 | 7 |
| | Smoothness (dry hair) | 8 | 7 | 6.5 | 6.8 | 6 |

Further examples are listed below:

| Ingredients | Ex. 1 | Ex. 2 | Ex. 3 | Ex. 4 |
|---|---|---|---|---|
| Polysorbate 60 | 1.2 | 1.1 | 0.9 | 1.0 |
| Polyquaternium-37¹ | 0.8 | 1.5 | 1.1 | 1.8 |
| Behentrimonium Chloride (70% active content)+ Dipropylene Glycol (30 % active content) | 0.45 | 0.5 | 0.3 | 1.0 |
| Dimethicone | | | 1.7 | 2 |
| Silicone Emulsion (Xiameter MEM-1352 Emulsion, Dow Corning) | 2.5 | | | |
| Silicone Emulsion (Xiameter MEM-1664 Emulsion, Dow Corning) | | 1.9 | | |
| C12-15 Alkyl Benzoate³ | 1.2 | 3.5 | 2.5 | 2 |
| Polyquaternium-4² | 0.8 | 0.9 | 0.6 | 0.7 |
| Sodium Benzoate | | 0.12 | 0.1 | |
| Sodium Salicylate | | 0.3 | 0.31 | |
| Phenoxyethanol | 0.6 | | | 0.7 |
| Benzethonium Chloride | 0.08 | | | |
| Perfume | q.s. | q.s. | q.s. | q.s. |
| Aqua | ad 100 | ad 100 | ad 100 | ad 100 |
| ¹Cosmedia® Ultragel 300 from BASF | | | | |
| ²Celquat® L-200 from AkzoNobel. | | | | |
| ³TEGOSOFT TN B from Evonik Industries | | | | |

## Claims

1. Cosmetic hair styling composition comprising in a cosmetically acceptable medium Polyquaternium-4, Polyquaternium-37 and C12-15 Alkyl Benzoate.

2. Cosmetic hair styling composition according to claim 1 **characterized in that** the concentration of Polyquaternium-4 in the cosmetic hair styling composition of the invention is in the range from 0.1% to 2% by weight and preferably from 0.5 to 1% by weight, calculated to the total weight of the composition.

3. Cosmetic hair styling composition according to any of the claims 1 to 2 **characterized in that** the Polyquaternium-37 is suspended in solid form in the cosmetic composition.

4. Cosmetic hair styling composition according to any of the claims 1 to 3 **characterized in that** the concentration of Polyquaternium-37 in the cosmetic hair styling composition is in the range from 0.1% to 2% by weight and preferably from 0.5 to 1.5% by weight, calculated to the total weight of the composition.

5. Cosmetic hair styling composition according to any of the claims 1 to 4 **characterized in that** the concentration of C12-C15 Alkyl Benzoate in the cosmetic hair styling composition of the invention is in the range from 0.1 % to 6% by weight and preferably from 1% to 3% by weight, calculated to the total weight of the composition.

6. Cosmetic hair styling composition according to any of the claims 1 to 5 **characterized in that** the compositions is an O/W-Emulsion.

7. Cosmetic hair styling composition according to any of the claims 1 to 6 **characterized in that** the cosmetic hair styling composition contains at least one emulsifier having an HLB value in the range from 13 to 17 and preferably from 15 to 16.

8. Cosmetic hair styling composition according to any of the claims 1 to 7 **characterized in that** the cosmetic hair styling composition contains sorbitan monostearate, ethoxylated with 20 mol EO, and/or sorbian monooleate, ethoxylated with 20 EO.

9. Cosmetic hair styling composition according to any of the claims 1 to 8 **characterized in that** the cosmetic hair styling further comprises a water insoluble, non-volatile silicone compound.

10. Cosmetic hair styling composition according to claim 9 **characterized in that** the cosmetic hair styling composition contains a linear, branches, cyclic or cyclic and branched dimethicone.

11. Cosmetic hair styling composition according to claim 9 **characterized in that** the cosmetic hair styling composition contains a silicone emulsion.

12. Cosmetic hair styling composition according to claim 11 **characterized in that** the silicone emulsion is therein characterized that the average diameter of the silicon particles is less than 1000 nm and preferably less than 600 nm.

13. Cosmetic hair styling composition according to any of the claims 11 to 12 **characterized in that** the silicone emulsion is therein characterized that it contains
(i) 30% to 70% by weight, calculated to the total weight of the emulsion, of at least one polyalkylsiloxane;
(ii) at least one nonionic emulsifier selected from alkoxylated C8 to C24 alcohols containing 18 to 30 ethylenoxid groups; and
(iii) at least one nonionic emulsifier selected from alkoxylated C8 to C24 alcohols containing 1 to 6 ethylenoxid groups.

14. Cosmetic hair styling composition according to any of the claims 1 to 13 **characterized in that** the cosmetic hair styling composition comprises at least one cationic surfactant.

15. Cosmetic hair styling composition according to claim 14 **characterized in that** the concentration of the cationic surfactant is in the range from 0.1% to 1.4% by weight, calculated to the total weight of the composition.

16. Use of a cosmetic hair styling composition according to any of the claims 1 to 15 to provide shine to the hair.

17. Use of a cosmetic hair styling composition according to any of the claims 1 to 15 to increase the smoothness of the hair.

## Patentansprüche

1. Kosmetische Haarstylingzusammensetzung, umfassend in einem kosmetisch unbedenklichen Medium Polyquaternium-4, Polyquaternium-37 und C12-15-Alkylbenzoat.

2. Kosmetische Haarstylingzusammensetzung nach Anspruch 1, **dadurch gekennzeichnet, dass** die Konzentration von Polyquaternium-4 in der kosmetischen Haarstylingzusammensetzung im Bereich von 0,1 bis 2 Gew.-% und vorzugsweise von 0,5 bis 1 Gew.-%, berechnet auf das Gesamtgewicht der Zusammensetzung, liegt.

3. Kosmetische Haarstylingzusammensetzung nach einem der Ansprüche 1 bis 2, **dadurch gekennzeichnet, dass** das Polyquaternium-37 in fester Form in der kosmetischen Zusammensetzung suspendiert ist.

4. Kosmetische Haarstylingzusammensetzung nach einem der Ansprüche 1 bis 3, **dadurch gekennzeichnet, dass** die Konzentration von Polyquaternium-37 in der kosmetischen Haarstylingzusammensetzung im Bereich von 0,1 bis 2 Gew.-% und vorzugsweise von 0,5 bis 1,5 Gew.-%, berechnet auf das Gesamtgewicht der Zusammensetzung, liegt.

5. Kosmetische Haarstylingzusammensetzung nach einem der Ansprüche 1 bis 4, **dadurch gekennzeichnet, dass** die Konzentration von C12-C15-Alkylbenzoat in der kosmetischen Haarstylingzusammensetzung der Erfindung im Bereich von 0,1 bis 6 Gew.-% und vorzugsweise von 1 bis 3 Gew.-%, berechnet auf das Gesamtgewicht der Zusammensetzung, liegt.

6. Kosmetische Haarstylingzusammensetzung nach einem der Ansprüche 1 bis 5, **dadurch gekennzeichnet, dass** es sich bei der Zusammensetzung um eine O/W-Emulsion handelt.

7. Kosmetische Haarstylingzusammensetzung nach einem der Ansprüche 1 bis 6, **dadurch gekennzeichnet, dass** die kosmetische Haarstylingzusammensetzung mindestens einen Emulgator mit einem HLB-Wert im Bereich von 13 bis 17 und vorzugsweise von 15 bis 16 enthält.

8. Kosmetische Haarstylingzusammensetzung nach einem der Ansprüche 1 bis 7, **dadurch gekennzeichnet, dass** die kosmetische Haarstylingzusammensetzung mit 20 mol EO ethoxyliertes Sorbitanmonostearat und/oder mit 20 EO ethoxyliertes Sorbitanmonooleat enthält.

9. Kosmetische Haarstylingzusammensetzung nach einem der Ansprüche 1 bis 8, **dadurch gekennzeichnet, dass** die kosmetische Haarstyling eine wasserunlösliche, nichtflüchtige Silikonverbindung umfasst.

10. Kosmetische Haarstylingzusammensetzung nach Anspruch 9, **dadurch gekennzeichnet, dass** die kosmetische Haarstylingzusammensetzung ein lineares, verzweigtes, cyclisches oder cyclisches unverzweigtes Dimethicon enthält.

11. Kosmetische Haarstylingzusammensetzung nach Anspruch 9, **dadurch gekennzeichnet, dass** die kosmetische Haarstylingzusammensetzung eine Silikonemulsion enthält.

12. Kosmetische Haarstylingzusammensetzung nach Anspruch 11, **dadurch gekennzeichnet, dass** die Silikonemulsion **dadurch gekennzeichnet ist, dass** der mittlere Durchmesser der Siliziumteilchen weniger als 1000 nm, vorzugsweise weniger als 600 nm beträgt.

13. Kosmetische Haarstylingzusammensetzung nach einem der Ansprüche 11 bis 12, **dadurch gekennzeichnet, dass** die Silikonemulsion **dadurch gekennzeichnet ist, dass** sie
(i) 30 bis 70 Gew.-%, berechnet auf das Gesamtgewicht der Emulsion, mindestens eines Polyalkylsiloxans;
(ii) mindestens einen nichtionischen Emulgator, der aus alkoxylierten C8- bis C24-Alkoholen mit 18 bis 30 Ethylenoxidgruppen ausgewählt ist; und
(iii) mindestens einen nichtionischen Emulgator, der aus alkoxylierten C8- bis C24-Alkoholen mit 1 bis 6 Ethylenoxidgruppen ausgewählt ist;
enthält.

14. Kosmetische Haarstylingzusammensetzung nach einem der Ansprüche 1 bis 13, **dadurch gekennzeichnet, dass** die kosmetische Haarstylingzusammensetzung mindestens ein kationisches Tensid umfasst.

15. Kosmetische Haarstylingzusammensetzung nach Anspruch 14, **dadurch gekennzeichnet, dass** die Konzentration des kationischen Tensids im Bereich von 0,1 bis 1,4 Gew.-%, berechnet auf das Gesamtgewicht der Zusammensetzung, liegt.

16. Verwendung einer kosmetischen Haarstylingzusammensetzung nach einem der Ansprüche 1 bis 15, um dem Haar Glanz zu verleihen.

17. Verwendung einer kosmetischen Haarstylingzusammensetzung nach einem der Ansprüche 1 bis 15, um die Glätte des Haars zu erhöhen.

## Revendications

1. Composition de coiffage capillaire cosmétique comprenant, dans un milieu acceptable sur le plan cosmétique, un polyquaternium-4, un polyquaternium-37 et un benzoate d'alkyle en C12 à 15.

2. Composition de coiffage capillaire cosmétique selon la revendication 1 **caractérisée en ce que** la concentration de polyquaternium-4 dans la composition de coiffage capillaire cosmétique de l'invention est dans la plage de 0,1 % à 2 % en poids et préférablement de 0,5 à 1 % en poids, calculée par rapport au poids total de la composition.

3. Composition de coiffage capillaire cosmétique selon l'une quelconque des revendications 1 et 2 **caractérisée en ce que** le polyquaternium-37 est en suspension sous forme solide dans la composition cosmétique.

4. Composition de coiffage capillaire cosmétique selon l'une quelconque des revendications 1 à 3 **caractérisée en ce que** la concentration de polyquaternium-37 dans la composition de coiffage capillaire cosmétique est dans la plage de 0,1 % à 2 % en poids et préférablement de 0,5 à 1,5 % en poids, calculée par rapport au poids total de la composition.

5. Composition de coiffage capillaire cosmétique selon l'une quelconque des revendications 1 à 4 **caractérisée en ce que** la concentration de benzoate d'alkyle en C12 à C15 dans la composition de coiffage capillaire cosmétique de l'invention est dans la plage de 0,1 % à 6 % en poids et préférablement de 1 % à 3 % en poids, calculée par rapport au poids total de la composition.

6. Composition de coiffage capillaire cosmétique selon l'une quelconque des revendications 1 à 5 **caractérisée en ce que** les compositions sont une émulsion huile/eau.

7. Composition de coiffage capillaire cosmétique selon l'une quelconque des revendications 1 à 6 **caractérisée en ce que** la composition de coiffage capillaire cosmétique contient au moins un émulsifiant possédant une valeur HLB dans la plage de 13 à 17 et préférablement de 15 à 16.

8. Composition de coiffage capillaire cosmétique selon l'une quelconque des revendications 1 à 7 **caractérisée en ce que** la composition de coiffage capillaire cosmétique contient du monostéarate de sorbitane, éthoxylé avec 20 moles d'EO, et/ou du monooléate de sorbitane, éthoxylé avec 20 EO.

9. Composition de coiffage capillaire cosmétique selon l'une quelconque des revendications 1 à 8 **caractérisée en ce que** le coiffage capillaire cosmétique comprend en outre un composé de type silicone insoluble dans l'eau, non volatil.

10. Composition de coiffage capillaire cosmétique selon la revendication 9 **caractérisée en ce que** la composition de coiffage capillaire cosmétique contient un diméthicone linéaire, ramifié, cyclique ou cyclique et ramifié.

11. Composition de coiffage capillaire cosmétique selon la revendication 9 **caractérisée en ce que** la composition de coiffage capillaire cosmétique contient une émulsion de silicone.

12. Composition de coiffage capillaire cosmétique selon la revendication 11 **caractérisée en ce que** l'émulsion de silicone est ici **caractérisée en ce que** le diamètre moyen des particules de silicium est inférieur à 1 000 nm et préférablement inférieur à 600 nm.

13. Composition de coiffage capillaire cosmétique selon l'une quelconque des revendications 11 et 12 **caractérisée en ce que** l'émulsion de silicone est ici **caractérisée en ce qu'**elle contient
(i) 30 % à 70 % en poids, calculé par rapport au poids total de l'émulsion, d'au moins un polyalkylsiloxane ;
(ii) au moins un émulsifiant non ionique choisi parmi les alcools en C8 à C24 alcoxylés contenant 18 à 30 groupes de type oxyde d'éthylène ; et
(iii) au moins un émulsifiant non ionique choisi parmi les alcools en C8 à C24 alcoxylés contenant 1 à 6 groupes de type oxyde d'éthylène.

14. Composition de coiffage capillaire cosmétique selon l'une quelconque des revendications 1 à 13 **caractérisée en ce que** la composition de coiffage capillaire cosmétique comprend au moins un tensioactif cationique.

15. Composition de coiffage capillaire cosmétique selon la revendication 14 **caractérisée en ce que** la concentration du tensioactif cationique est dans la plage de 0,1 % à 1,4 % en poids, calculée par rapport au poids total de la composition.

16. Utilisation d'une composition de coiffage capillaire cosmétique selon l'une quelconque des revendications 1 à 15 pour apporter du brillant aux cheveux.

17. Utilisation d'une composition de coiffage capillaire cosmétique selon l'une quelconque des revendications 1 à 15 pour augmenter la douceur des cheveux.
